# EUROPEAN PATENT SPECIFICATION

(11) **EP 2 775 874 B1**
(45) Date of publication and mention of the grant of the patent: **10.01.2018**
(21) Application number: 12806672.7
(22) Date of filing: 08.11.2012
(51) Int. Cl.: A41G 5/00, A61K 38/06, A61P 17/00, A61P 17/06

(54) **PHARMACEUTICAL COMPOSITION FOR THE PREVENTION AND THE TREATMENT OF DEGENERATIVE SKIN DISORDERS ESPECIALLY CAUSED BY IONIZING RADIATIONS**
PHARMAZEUTISCHE ZUSAMMENSETZUNG ZUR VORBEUGUNG UND BEHANDLUNG VON DEGENERATIVEN HAUTERKRANKUNGEN INSBESONDERE DURCH IONISIERENDE STRAHLUNG
COMPOSITION PHARMACEUTIQUE POUR LA PRÉVENTION ET LE TRAITEMENT DE TROUBLES DÉGÉNÉRATIFS DE LA PEAU EN PARTICULIER PROVOQUÉS PAR DES RAYONNEMENTS IONISANTS

(30) Priority: 08.11.2011 IT PO20110021
(43) Date of publication of application: 17.09.2014
(73) Proprietor: Solosale S.r.l., 50132 Firenze (IT)
(72) Inventor: FEDELI, Enrichetta, I-50141 Firenze (IT); SERENSI, Ilaria, I-50135 Firenze (IT)
(74) Representative: Martini, Riccardo
(86) International application number: PCT/IB2012/056269
(87) International publication number: WO 2013/068964

(56) References cited:
- WO-A1-2004/010968
- WO-A1-2011/081716
- WO-A2-2009/047728
- JP-A- 63 267 711
- US-A1- 2011 160 144
- FALCK ET AL: "Preparation of N-<t>Boc l-glutathione dimethyl and di-tert-butyl esters: Versatile synthetic building blocks", BIOORGANIC & MEDICINAL CHEMISTRY, PERGAMON, GB, vol. 15, no. 2, 12 December 2006 (2006-12-12), pages 1062-1066, XP005882825, ISSN: 0968-0896, DOI: 10.1016/J.BMC.2006.10.022
- Alan Katritzky ET AL: "Efficient and Selective Syntheses of S-Acyl and N-Acyl Glutathiones", SYNLETT, vol. 2010, no. 09, 16 April 2010 (2010-04-16), pages 1337-1340, XP055281907, DE ISSN: 0936-5214, DOI: 10.1055/s-0029-1219837

## Description

### Technical Field

The present invention relates to a pharmaceutical composition containing S-acyl-derivatives of glutathione with unsaturated fatty acids, as defined in the claims, usable for the prevention and treatment of cutaneous degenerative disorders especially caused by ionizing radiation, in particular by ultraviolet radiation from the sun or artificial.

### Background Art

It is well known that free radicals play an important role in aging and that our body has defence strategies against free radicals represented by a set of substances and antioxidant mechanisms, such as vitamins A, E and C and the enzymes superoxide dismutase and catalase. The defence represented by glutathione is the most effective, due to its high intracellular concentration.

Pharmacological research has tried to slow down the effects of time on the aging of tissues by restoring, at least temporarily, the lack of the key substances required by the body through the synergistic combination of topical treatments and specific nutritional supplements, for example through the supplying of antioxidant substances able to limit the damage caused by free radicals.

Recent studies have also shown that in many degenerative diseases such as diabetes, hepatitis, ulcerative colitis, Crohn's disease, renal dysfunction, myocardial ischemia, adult respiratory distress syndrome and Alzheimer's disease, the loss of cells is related to oxidative stress and to a reduction in the levels of intracellular substances with antioxidant activity, notably reduced glutathione (GSH).

Glutathione plays a key role in the cellular defense by acing as a powerful antioxidant due to its high capacity of electron donor combined with its high intracellular concentration. In order to maintain or restore an adequate level of cellular antioxidant defenses in tissues affected by oxidative stress, several therapeutic strategies have been developed aimed at increasing the levels of GSH through the intake, via food or drugs, of glutathione precursors or mimetics. In particular, glutathione or derivatives of glutathione or its precursor cysteine, such as N-acetyl glutathione and N-acetyl cysteine, can be found in many commercially available preparations.

PCT application WO2009047728 describes the use of S-acyl derivatives of glutathione. These thioesters, in which the lipid moiety is made up of long chain fatty acids, are used in the preparation of pharmaceutical compositions for the prevention and treatment of degenerative diseases associated with a decreased levels of intracellular glutathione, such as Huntington's chorea and Alzheimer's disease.

Common causes of oxidative stress related to the reduction of intracellular glutathione levels and to ageing include physical and environmental agents.

The skin, in particular, is subjected to cellular aging processes, due to the exposure to the ultraviolet component of solar radiation, ultraviolet radiation from artificial sources such as tanning lamps and other ionizing radiation - X-, gamma-, beta-, alpha-rays - from both natural and artificial sources.

Recently, polyunsaturated fatty acids (for example alpha-and gamma-linoleic acids, abundantly present in the linseed oil) have ben demonstrated to exert a stimulating action on the hydrating macrostructures of the dermis leading to an anti-aging effect. Numerous studies have also described the anti-aging effects of compounds acting as radical scavengers. These substances elicit antiradicals activity and usually are of natural origin such as flavonoids of gingko biloba, polyphenols from Hamamelidis cortex, resveratrol, lycopenes and many others that also exert a protective action on the skin microcirculation. Nonetheless, the most effective anti-free radicals are antioxidant vitamins and selenium, which the body is unable to synthesize directly but must necessarily intake through the diet.

It is well known that the exposure of the skin to ultraviolet component of solar radiation, or to ultraviolet light produced by artificial tanning lamps, significantly increases the likelihood of onset of skin diseases, such as melanoma, hyperkeratosis, angioectasias, fibroids, etc., as well as determining unsightly skin alterations related to photo-induced aging processes. Currently the protection from skin degeneration processes induced by exposure to sunlight or to artificial ultraviolet light is essentially based on the use of physical barriers, such as garments, which prevent the interaction of ionizing radiation with the skin, or on the application on epidermis of cosmetics containing substances able to filter the ultraviolet radiation. However the garments, apart from very special cases, protect only a fraction of the total skin surface, whereas the use of sunscreens may be disadvantageous in cases where the stimulation of melatonin synthesis by melanocytes is desirable for aesthetic purposes. On the other hand, the exposure of the skin to solar radiation is accompanied by other desirable effects, such as stimulation of the synthesis of vitamin D through the conversion of 7-dehydrocholesterol in cholecalciferol and the skin tanning effect.

It is so strongly felt the need for solutions that allow the skin to be moderately exposed to sunlight or to artificial ultraviolet light without producing unwanted side effects such as cellular aging or skin degenerative diseases.

### Disclosure of Invention

In general, the object of the present invention is to provide a pharmaceutical composition to be used for the prevention and treatment of cellular degenerative processes of the skin caused by physical and environmental agents.

More specifically, it is a purpose of the invention to provide a pharmaceutical composition, containing S-acyl derivatives of glutathione with unsaturated fatty acids, for the prevention and treatment of degenerative skin disorders caused by exposure to ultraviolet radiation or other ionizing radiation.

In particular, object of the invention is to provide a pharmaceutical formulation containing thioesters of glutathione with long-chain polyunsaturated fatty acids as defined in the claims and the use of said formulation to facilitate the delivery of substances with antioxidant activity, including the reduced glutathione, within the skin cells. Also disclosed is a new method for the synthesis of S-acyl derivatives of glutathione, in particular thioesters of glutathione with unsaturated long chain fatty acids, especially suitable for producing pharmaceutical compositions for the prevention and treatment of degenerative skin states and cellular aging caused by skin exposure to ionizing radiation, in particular to ultraviolet radiation from natural or artificial sources.

The above purposes are achieved by means of a composition in accordance with the accompanying claims.

### Best Mode for Carrying Out the Invention

According to the invention are provided thioester derivatives of glutathione having formula 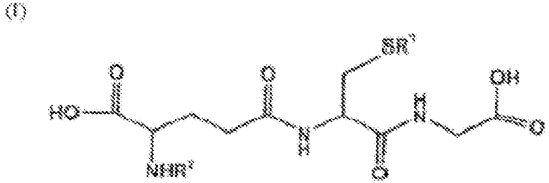 wherein R¹ is selected from radicals of linolenic, arachidonic, eicosapentaenoic and docosahexaenoic acids and R² is hydrogen or an acyl group aliphatic or aromatic, preferably aliphatic, and most preferably a C₁₈-C₂₄ polyunsaturated acyl group. In a particularly preferred mode for carrying out the invention, R₁ and R₂ are radicals selected from those of linolenic acid, eicosapentaenoic acid, docosahexaenoic acid and arachidonic acid.

Here below it is shown the structural formula of the thioester of glutathione with linolenic acid.

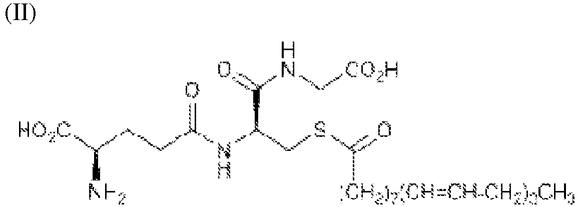

Due to their lipophilic nature, S-acyl and S, N-diacyl derivatives of glutathione according to the invention easily cross the cell membrane and within the cell are enzymatically hydrolyzed by esterases thus releasing reduced glutathione and free carboxylic acids. The ability to cross cell membranes is to be ascribed to the presence of the apolar hydrocarbon chain of the acyl groups linked to the thiol group or to thiol and amino groups of glutathione.

A pharmaceutical composition in accordance with the present invention comprises a mixture of active substances with antioxidant activity, at least one of them being represented by a thioester of glutathione with a long chain, polyunsaturated, fatty acid as specified above, together with agents endowed with different activities such as emulsifiers, preservatives, pigments, flavors, humectants, etc. In particular, the thioester of glutathione with a long chain polyunsaturated fatty acid is contained in the composition at a concentration ranging between 1 µM and 100 mM, preferably between 50 µM and 5 mM.

Two examples of such a pharmaceutical composition are given here following. It is well understood that these examples represent only two of the possible applications of the present invention and are not limitative of the same.

### Example 1

linolenoyl-S-glutathione
vitamin C
vitamin A
vitamin E
selenium

### Example 2

eicosapentaenoyl-S-glutathione
glycolic acid
vitamin C
vitamin E
resveratrol
hyaluronic acid
lecithin
triacylglycerols

The effectiveness of the pharmaceutical formulation described in the present invention is demonstrated by results obtained in cultured fibroblasts exposed to ultraviolet radiation. The incubation of these cells for 3 h with the formulation containing 100 µM S-acyl-glutathione significantly increased the intracellular content of reduced glutathione and resulted in a significantly higher survival compared to control fibroblasts, as shown in Table I:

**Table I**

| Study model | acyl-S-glutathione | intracellular GSH, % compared to controls (3 h after incubation) | cell survival, % (24 h after end of irradiation) |
|---|---|---|---|
| | | | |
| Cultured skin fibroblasts exposed to UV rays | absent | 30 | 10 |
| Cultured skin fibroblasts exposed to UV rays | oleyl-S-glutathione | 85 | 35 |
| Cultured skin fibroblasts exposed to UV rays | linoleyl-S-glutathione | 110 | 63 |
| Cultured skin fibroblasts exposed to UV rays | linolenoyl-S-glutathione | 115 | 97 |
| Cultured skin fibroblasts exposed to UV rays | S,N-di-linolenoyl-S- glutathione | 113 | 98 |
| Cultured skin fibroblasts exposed to UV rays | arachidonoyl-S-glutathione | 115 | 100 |
| | | | |
| cultured skin fibroblasts (control) | absent | 100 | 100 |

The effectiveness of the formulation for determining an increase of intracellular GSH and of cell survival following exposure to ultraviolet radiation was as greater as higher was the number of carbon atoms and the degree of unsaturation of the fatty acid linked to glutathione, linolenic and arachidonic acids being apparently the most effective. Similar experiments carried out with the use of eicosapentaenoic acid and docosahexaenoic acid provided results similar to those obtained with linolenic and arachidonic acids. The maximum efficacy of the formulation was observed when 100 µM S-acyl-glutathione conjugate was used. Higher concentrations, up to 50 mM, of conjugate did not proved to be more efficient in producing the observed effects. Lower concentrations showed a proportionally smaller activity than that displayed by formulations containing 100 µM conjugate, whereas concentrations below 0.5 µM demonstrated not to possess any activity.

Considering that the average concentration of glutathione in this kind of cells is about 2 mM, the increase of intracellular glutathione concentration caused by the treatment with glutathione thioesters at these low concentrations is actually surprising and hardly attributable to the simple internalization of glutathione thioesters by the cells, but rather to a stimulation of the endogenous synthesis of glutathione or to a different mechanism of regulation of gene expression affecting the turnover of enzymes involved in glutathione metabolism.

Similar experiments carried out using S,N-diacyl glutathione derivatives, in which both fatty acids were polyunsaturated and whose carbon atoms ranged between 18 and 24, showed similar efficiency to that elicited by S-acyl glutathione derivatives.

Similar experiments carry out using antioxidant compounds other than S-acyl glutathione derivatives, such as ascorbic acid, vitamin E, Trolox ®, resveratrol, at the same concentrations did not produce significant effects, in terms of effectiveness, for the purposes of the present invention.

In other experiments, the formulation described in Example 2 was applied on the skin of adult subjects who had issued informed consent. After one hour of irradiation with ultraviolet lamp, the degree of erythema observed, measured by spectro-reflectometry, was not significantly different as compared to the measured values in the same area of skin, prior to the treatment. Otherwise, the difference was highly significant in control subjects, on whose skin the formulation had not been applied.

These results justify the possible use of S-acyl derivatives of glutathione according to the present invention for the preparation of pharmaceutical compositions useful for the prevention and treatment of degenerative skin states and cell aging caused by skin exposure to ionizing radiation, in particular ultraviolet radiation from natural or artificial sources.

Said pharmaceutical compositions comprise, as active ingredient, a compound of formula (I) or a pharmaceutically acceptable salt thereof, alone or in association with a pharmaceutically acceptable excipient such as a carrier or a diluent.

The compositions are preferably prepared in a form suitable for oral administration or for injection. The pharmaceutically acceptable excipients that are mixed with the active compound or salts of such compound, to prepare the compositions according to the invention are well known per se and their choice depends, inter alia, by the method prescribed for the administration of the compositions.

The compositions for oral administration may take the form of tablets, retard tablets, sublingual tablets, capsules and the like or elixirs, syrups or suspensions, all containing a compound according to the invention. Compositions for injection may be prepared from soluble salts, which may be dissolved in an appropriate fluid for parenteral injection. Compositions for local use may take the form of creams, gels, serums, lotions and the like, all containing a compound according to the invention. All preparations mentioned herein may be produced by methods well known in the field.

S-acyl derivatives of glutathione according to the invention can be synthesized by reacting glutathione with benzotriazole derivatives of fatty acids.

Methods for the synthesis biochemistry or chemistry of thioesters of glutathione are described in literature (Free Radical Biology & Medicine 44 (2008) 1624-1636; Synlett 2010 (9): 1337-1340; Chem. Res Toxicol. 14:1033-1040, 2001; Bioorg. Med Chem. 15:1062-1066, 2007) and in WO2009047728.

The methods available to date are however characterized by high cost or very low yields or difficulties in purification of the thioester or produce salts of the thioester with anions not usable for the purpose of the present invention, and are therefore not suitable for the industrial synthesis of thioesters of glutathione with long-chain fatty acids usable for the purpose of the present invention. Katritzky et al. (Synlett 2010 (9): 1337-1340) describes the synthesis of glutathione thioesters with carboxylic aromatic compounds or with other carboxylic compounds, but not with long-chain fatty acids.

The method here described is optimal for industrial production of glutathione with thioesters of fatty acids with a number of carbon atoms between 12 and 24, either saturated or unsaturated or polyunsaturated, and ensures the production of products with high degree of purity, higher then 98%, using reagents easily available and of low cost. In addition, a significant advantage of the organic synthesis here described is represented by the yield, i.e. the amount of product obtainable compared to the quantity of reagents used, which appears to be between 60 and 99% depending on the nature of fatty acid.

Here following the synthesis is described in a schematic way:
A) 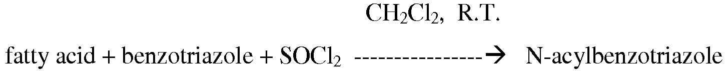
B)

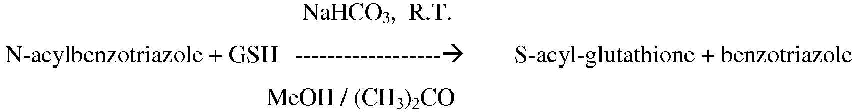

The synthesis in conformity of the present invention can be carried out by experts in the art in different ways, however it is particularly advantageous to implement the following sequence of phases, in order to obtain the product with the purity and yield mentioned above:
1) Synthesis of N-acylbenzotriazole by reaction between benzotriazole, thionyl chloride and fatty acid in methylene chloride.
2) Synthesis at room temperature of S-acyl-glutathione by addition of two equivalents of HCO³⁻ to one equivalent of N-acylbenzotriazole and one equivalent of glutathione, in methanol/acetone.
3) pH adjustment, preferably with acetic acid or phosphoric acid, to values between 4 and 2.5 and filtration of the precipitate.
4) Washing the precipitate with water and organic solvent, preferably ethyl acetate.

## Claims

1. Pharmaceutical composition for use in the prevention or treatment of skin degenerative disorders and cellular aging caused by skin exposure to ionizing radiation, in particular to solar or artificial ultraviolet radiation, comprising at least one thioester of glutathione with a fatty acid as an active ingredient, having the following formula 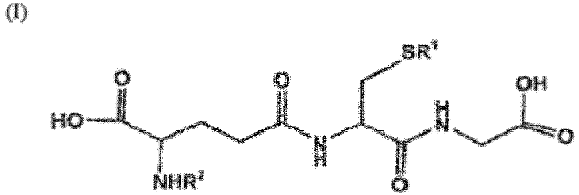 wherein R1 is selected from radicals of linolenic, arachidonic, eicosapentaenoic and docosahexaenoic acids and R2 is hydrogen or an aliphatic or aromatic acyl group.

2. A pharmaceutical composition for use according to claim 1, wherein R2, is a C18-C24 polyunsaturated group.

3. A pharmaceutical composition for use according to any one of the preceding claims, in which the thioester of glutathione is contained in a concentration of between 1 µM and 100 mM, preferably between 50 µM and 5 mM, 100 µM being the optimal concentration.

4. A pharmaceutical composition for use according to any one of the preceding claims comprising, as an active ingredient, a compound of formula (I) or a pharmaceutically acceptable salt thereof alone or in association with a pharmaceutically acceptable excipient such as a carrier or a diluent.

5. A pharmaceutical composition for use according to any one of the preceding claims, prepared in a form suitable for oral administration or for injection or locally applicable.

## Patentansprüche

1. Pharmazeutische Zusammensetzung zur Verwendung bei der Vorbeugung oder Behandlung von degenerativen Hauterkrankungen und Zellalterung, die durch Exposition der Haut gegenüber ionisierender Strahlung, insbesondere Sonnen- oder künstlicher Ultraviolettstrahlung, verursacht werden, umfassend mindestens einen Thioester aus Glutathion mit einer Fettsäure als Wirkstoff, der folgende Formel (I) aufweist: 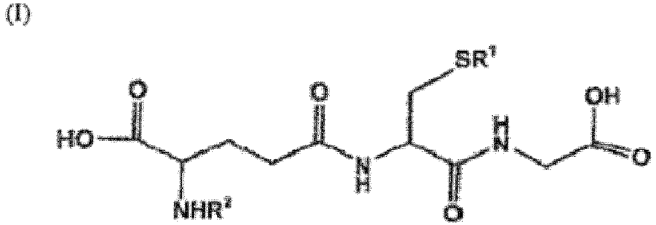 wobei R¹ ausgewählt wird aus Radikalen der Linolen-, Arachidon-, Eicosapentaen- und Docosahexaensäure und R² Wasserstoff oder eine aliphatische oder aromatische Acylgruppe ist.

2. Pharmazeutische Zusammensetzung zur Verwendung nach Anspruch 1, wobei R² eine mehrfach ungesättigte C₁₈-C₂₄-Gruppe ist.

3. Pharmazeutische Zusammensetzung zur Verwendung nach einem der vorstehenden Ansprüche, bei der der Thioester aus Glutathion in einer Konzentration von 1 µM bis 100 mM, vorzugsweise von 50 µM bis 5 mM enthalten ist, wobei die optimale Konzentration 100 µM beträgt.

4. Pharmazeutische Zusammensetzung zur Verwendung nach einem der vorstehenden Ansprüche, die als Wirkstoff eine Verbindung der Formel (I) oder ein pharmazeutisch annehmbares Salz davon allein oder in Kombination mit einem pharmazeutisch annehmbaren Hilfsstoff wie einem Trägerstoff oder einem Verdünnungsmittel umfasst.

5. Pharmazeutische Zusammensetzung zur Verwendung nach einem der vorstehenden Ansprüche, die in einer zur oralen Verabreichung oder zur Injektion oder zur lokalen Anwendung geeigneten Form zubereitet wird.

## Revendications

1. Composition pharmaceutique pour une utilisation dans la prévention ou le traitement de troubles dégénératifs de la peau et du vieillissement cellulaire causés par l'exposition de la peau à un rayonnement ionisant, en particulier un rayonnement ultraviolet solaire ou artificiel, comprenant au moins un thioester de glutathion avec un acide gras comme ingrédient actif, ayant la formule suivante 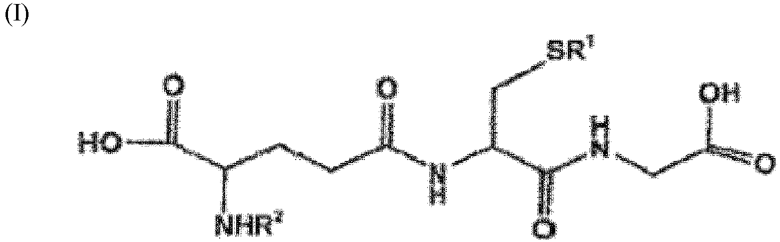 dans laquelle R¹ est choisi parmi des radicaux d'acides linolénique, arachidonique, eicosapentaénoïque et docosahexaénoïque et R² est de l'hydrogène ou un groupe acyle aliphatique ou aromatique.

2. Composition pharmaceutique pour une utilisation selon la revendication 1, dans laquelle R² est un groupe polyinsaturé en C₁₈-C₂₄.

3. Composition pharmaceutique pour une utilisation selon l'une quelconque des revendications précédentes, dans laquelle le thioester de glutathion est compris à une concentration comprise entre 1 µM et 100 mM, de préférence entre 50 µM et 5 mM, 100 µM étant la concentration optimale.

4. Composition pharmaceutique pour une utilisation selon l'une quelconque des revendications précédentes, comprenant, en tant qu'ingrédient actif, un composé de formule (I) ou un sel pharmaceutiquement acceptable de celui-ci, seul ou en association avec un excipient pharmaceutiquement acceptable tel qu'un véhicule ou un diluant.

5. Composition pharmaceutique pour une utilisation selon l'une quelconque des revendications précédentes, préparée sous une forme appropriée pour une administration orale ou pour une injection ou applicable localement.
